(19) Europäisches Patentamt
European Patent Office
Office européen des brevets

(11) Veröffentlichungsnummer: **0 056 114**
**A1**

# (12) EUROPÄISCHE PATENTANMELDUNG

(21) Anmeldenummer: **81110105.4**

(22) Anmeldetag: **03.12.81**

(51) Int. Cl.³: **C 07 D 213/74**, C 07 D 401/12, A 61 K 31/44 // C07D213/75

(30) Priorität: **10.01.81 DE 3100516**

(43) Veröffentlichungstag der Anmeldung: **21.07.82**
**Patentblatt 82/29**

(84) Benannte Vertragsstaaten: **AT BE CH DE FR GB IT LI LU NL SE**

(71) Anmelder: **C.H. BOEHRINGER SOHN, Postfach 200, D-6507 Ingelheim am Rhein (DE)**

(84) Benannte Vertragsstaaten: **BE CH DE FR IT LI LU NL SE AT**

(71) Anmelder: **Boehringer Ingelheim International G.m.b.H, D-6507 Ingelheim am Rhein (DE)**

(84) Benannte Vertragsstaaten: **GB**

(72) Erfinder: **Schromm, Kurt, Dr., In der Dörrwiese 35, D-6507 Ingelheim am Rhein (DE)**
Erfinder: **Mentrup, Anton, Dr., Steinernstrasse 25, D-6503 Mainz-Kastel (DE)**
Erfinder: **Renth, Ernst-Otto, Dr., Frankenstrasse 11, D-6507 Ingelheim am Rhein (DE)**
Erfinder: **Fügner, Armin, Dr., Im Hippel 31, D-6535 Gau-Algesheim (DE)**

(54) **Pyridylaminobenzoesäuren, ihre Herstellung und Verwendung.**

(57) Es wurden Verbindungen der Formel

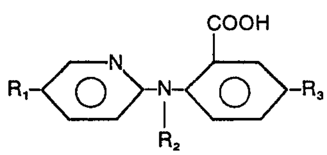

beschrieben,

in der $R_1$ eine $(CH_3)_2CH$-, $C_2H_5$- $CH(CH_3)$- oder $(C_2H_5)_2CH$-Gruppe, $R_2$ Wasserstoff, Formyl oder Acetyl und $R_3$ Carboxyl oder Tetrazol-5-yl bedeutet, sowie ihre Salze, die Herstellung dieser Verbindungen und ihre Verwendung. Die Verbindungen zeigen vor allem antiallergische Wirkung und können daher besonders bei der Behandlung und Prophylaxe allergischer Krankheiten angewendet werden.

EP 0 056 114 A1

COMPLETE DOCUMENT

ACTORUM AG

Die Erfindung betrifft neue Pyridylaminobenzoesäuren der Formel

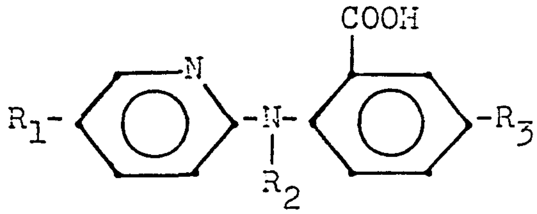

(I),

in der

$R_1$ für eine der Gruppen

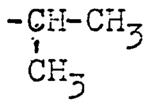

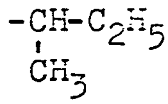

oder

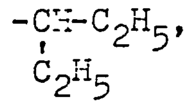

$R_2$ für Wasserstoff, -CHO oder $-CO-CH_3$

und

$R_3$ für -COOH oder

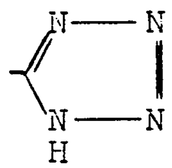

steht,

sowie ihre Salze mit anorganischen oder organischen Basen.

Die neuen Verbindungen sind für therapeutische Zwecke verwendbar und können auch als Zwischenprodukte für die Herstellung von pharmazeutischen Wirkstoffen und Präparaten dienen. Hervorzuheben ist die starke antiallergische Wirkung der Verbindungen der Formel I.

Aus der deutschen Offenlegungsschrift 27 35 919 sind verwandte Verbindungen bekannt, beispielsweise die Verbindung der Formel

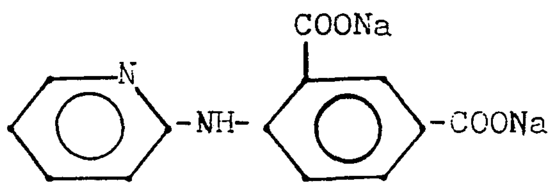

(A)

Die erfindungsgemäßen Verbindungen zeigen jedoch eine überraschend hohe Überlegenheit in der antiallergischen Wirkung. Beispielsweise liegt die $ED_{50}$ [mg/kg] an der Ratte im PCA-Test für die erfindungsgemäße Verbindung

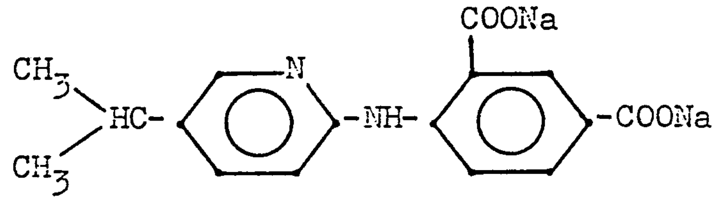

(B)

bei oraler Substanzgabe ca. 100-mal niedriger als für die Verbindung A.

Die Verbindungen der Formel I werden nach üblichen Verfahren hergestellt.

1. Man behandelt eine Verbindung der Formel

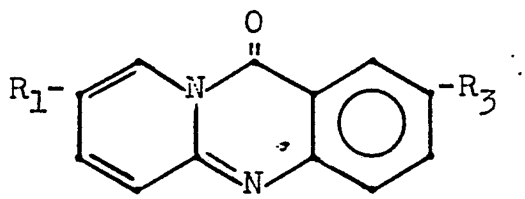

(II)

in der $R_1$ und $R_3$ die obige Bedeutung haben, mit basischen Stoffen, z.B. Alkalilaugen, etwa wäßriger Natronlauge. Gegebenenfalls wird anschließend die Aminogruppe nach üblichen Methoden formyliert oder acetyliert.
Die Ringöffnung kann bei niedrigen Temperaturen erfolgen, wird jedoch zweckmäßig unter Erwärmen, gewünschtenfalls bis zur Siedetemperatur des Reaktionsgemischs, durchgeführt.

2. Man wandelt in einer Verbindung der Formel

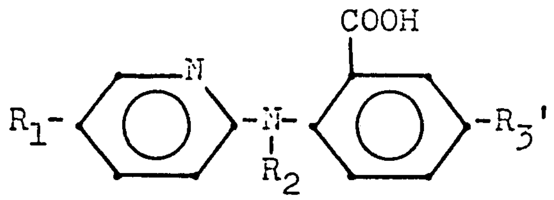 (III),

in der $R_1$ und $R_2$ die obige Bedeutung haben und $R_3'$ für eine Vorstufe von $R_3$ steht, die Gruppe $R_3'$ in die Gruppe $R_3$ um.

$R_3'$ kann insbesondere eine Estergruppe -COOR oder eine Säureamidgruppe -CONR'R" oder die CN-Gruppe darstellen. Durch Hydrolyse, insbesondere mit Basen, erhält man Verbindungen der Formel I. Aus Verbindungen der Formel III mit $R_3'$ gleich CN erhält man durch Azid-Addition solche Endprodukte, in denen $R_3$ den Tetrazol-5-yl-Rest bedeutet.

Bei den Estergruppen kann R den Kohlenwasserstoffteil eines beliebigen Alkohols bedeuten, der unter den Reaktionsbedingungen ausreichend beständig ist; jedoch bedeutet R bevorzugt einen niederen aliphatischen Kohlenwasserstoffrest oder einen gegebenenfalls substituierten Benzylrest.

Auch R' und R", die gleich oder verschieden sein können, können beliebige, unter den Reaktionsbedingungen ausreichend stabile, gegebenenfalls substituierte Kohlenwasserstoffreste bedeuten. Bevorzugt stehen sie für Wasserstoff, niedere aliphatische Reste oder gegebenenfalls substituierte Benzylreste.

Die Umwandlung der Gruppe $R_3'$ in $R_3$ erfolgt im allgemeinen bei Temperaturen zwischen Raumtemperatur und der Siedetemperatur des Reaktionsgemischs. Die Hydrolyse wird bevorzugt mit wäßrigen Alkalilaugen durchgeführt. Auch die Verwendung bzw. Mitverwendung von organischen Lösungsmitteln, die unter den Reaktionsbedingungen ausreichend beständig sind, ist möglich.

3. Man spaltet aus einer Verbindung der Formel

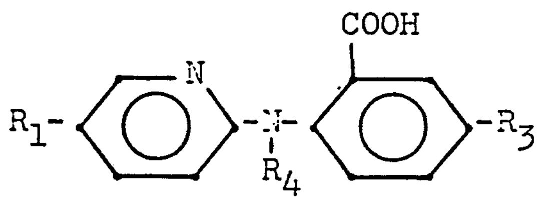

(IV),

in der $R_4$ einen von der Aminogruppe abspaltbaren Rest bedeutet, diesen Rest nach üblichen Methoden ab. $R_4$ steht vorzugsweise für einen Carbonsäurerest, insbesondere den Rest einer niederen aliphatischen Carbonsäure, oder einen gegebenenfalls substituierten Benzylrest. Je nach seiner Art wird der Rest $R_4$ hydrolytisch, insbesondere mit Basen, oder hydrogenolytisch in Gegenwart von Hydrierungskatalysatoren entfernt. Man erhält Verbindungen I mit $R_2$ gleich H.

Die Ausgangsstoffe der Formeln II bis IV können als freie Säuren oder als Salze für die erfindungsgemäßen Umsetzungen verwendet werden.

Die Ausgangsstoffe werden nach üblichen Methoden erhalten.

Die erfindungsgemäßen neuen Verbindungen sind therapeutisch verwendbar oder dienen als Zwischenprodukte für die Herstellung von Arzneistoffen. Hervorzuheben ist ihre antiallergische Wirkung. Diese kann für die Prophylaxe und Behandlung von allergischen Krankheiten wie Asthma oder auch bei Heufieber, Conjunctivitis, Urticaria, Ekzemen, atopischer Dermatitis ausgenutzt werden. Die Verbindungen zeigen außerdem muskelrelaxierende, bronchodilatorische und vasodilatorische Wirkung. Bei der wichtigsten Anwendung, der Asthma-Propylaxe, sind als Vorteile gegenüber dem Handelsprodukt Cromoglycinsäure die längere Wirkungsdauer und vor allem die orale Wirksamkeit zu nennen. Für die Anwendung werden die erfindungsgemäßen Verbindungen in üblicherweise mit Hilfs- und Trägerstoffen zu gebräuchlichen galenischen Zubereitungen verarbeitet, z.B. zu Kapseln, Tabletten, Dragées, Lösungen, Suspensionen für die orale Anwendung; zu Aerosolen für die pulmonale Gabe; zu sterilen isotonischen wäßrigen Lösungen für die parenterale Anwendung und zu Cremes, Salben, Lotionen, Emulsionen oder Sprays für die lokale Applikation. Die Einzeldosis hängt, außer von der Wirksubstanz, von der Indikation (z.B. der Beschaffenheit des allergischen Zustandes) und der Anwendungsweise ab. Im allgemeinen beträgt die Dosis pro kg Körpergewicht bei der pulmonalen Anwendung etwa 1 - 50 µg, bei der intravenösen Anwendung etwa 10 - 1000 µg, bei der oralen Anwendung etwa 0,05 bis 5 mg. Nasal oder okular werden etwa 0,02 bis 5 mg angewendet.

Beispiele für die pharmazeutischen Präparate mit erfindungsgemäßen Wirkstoffen:

Tabletten

Zusammensetzung:

| | |
|---|---|
| a) 4-(5-Isopropylpyridyl-2-amino)isophthalsäure-dinatriumsalz | 0,010 g |
| Stearinsäure | 0,001 g |
| Traubenzucker | 0,189 g |
| | 0,200 g |

| | |
|---|---|
| b) 2-(5-Isopropylpyridyl-2-amino)-5-(1H-tetrazol-5-yl)-benzoesäure-dinatriumsalz | 0,020 g |
| Stearinsäure | 0,002 g |
| Traubenzucker | 0,228 g |
| | 0,250 g |

Die Bestandteile werden in üblicher Weise verarbeitet und zu Tabletten von 200 mg verpreßt.

Salbe

Zusammensetzung:

| | | |
|---|---|---|
| Wirkstoff gemäß der Erfindung | | 0,200 g |
| rauchende Salzsäure | | 0,010 g |
| Natriumpyrosulfit | | 0,050 g |
| Gemisch aus gleichen Teilen Cetylalkohol und Stearylalkohol | | 20,000 g |
| weiße Vaseline | | 5,000 g |
| künstliches Bergamotteöl | | 0,075 g |
| destilliertes Wasser | ad | 100,000 g |

Die Bestandteile werden in üblicher Weise zu einer Salbe verarbeitet.

Inhalationsaerosol

Zusammensetzung:

| | |
|---|---|
| Wirkstoff gemäß der Erfindung | 0,5 Gew.-% |
| Sojalezithin | 0,2 Gew.-% |
| Treibgasmischung (Frigen 11,12 und 114) | 99,3 Gew.-% |

Die Zubereitung wird vorzugsweise in Aerosolbehälter mit Dosierventil abgefüllt. Der einzelne Hub wird so bemessen, daß eine Dosis von 0,5 mg abgegeben wird.

Ampullen (Injektionslösungen)

Zusammensetzung:

| | |
|---|---|
| Wirkstoff gemäß der Erfindung | 0,50 Gew.-% |
| Natriumpyrosulfit | 0,10 Gew.-% |
| Dinatriumsalz der Ethylendiamintetraessigsäure | 0,05 Gew.-% |
| Natriumchlorid | 0,85 Gew.-% |
| doppelt destilliertes Wasser | 98,50 Gew.-% |

Wirkstoff und Hilfsstoffe werden in einer ausreichenden Menge Wasser gelöst und mit der notwendigen Menge Wasser auf die angegebene Konzentration gebracht. Die Lösung wird filtriert und unter aseptischen Bedingungen in 1 ml-Ampullen abgefüllt. Zuletzt werden die Ampullen sterilisiert und verschlossen. Jede Ampulle enthält 5 mg Wirkstoff.

Für die Anwendung als Aerosol können die erfindungsgemäßen Wirkstoffe auch in mikronisierter Form (Teilchengröße im wesentlichen etwa 2 - 6 $\mu$m), gegebenenfalls unter Zusatz mikronisierter Trägerstoffe, etwa Lactose, in Hartgelatine-Kapseln gefüllt werden. Zur Applikation dienen übliche Geräte für die Pulverinhalation.

In jede Kapsel werden z.B. 0,2 bis 5 mg Wirkstoff und 0 bis 40 mg Lactose eingefüllt.

Die Herstellung der neuen Verbindungen ist in den nachstehenden Beispielen näher erläutert.

Zur zusätzlichen Charakterisierung wurden UV-Spektren von den Verbindungen aufgenommen. Es wurden die langwelligen Absorptionsbanden ausgewählt und die Absorptionen im Maximum als $\lambda$max [nm] mit den dazugehörigen molaren logarithmischen Extinktionskoeffizient log. $\varepsilon$ angegeben.

Beispiel 1

4-(5-Isopropylpyridyl-2-amino)isophthalsäure-dinatriumsalz

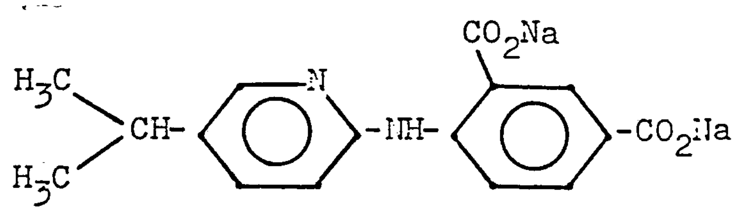

2,0 g 8-Isopropyl-11-oxo-11-H-pyridol[2,1-b]chinazolin-2-carbonsäure werden mit 14,2 ml normaler Natronlauge 1 Stunde auf dem Wasserbade erhitzt. Es entsteht eine klare Lösung. Danach wird das Wasser mit Toluol azeotrop abdestilliert. Die Kristalle werden in Äther aufgeschlämmt, abgesaugt und im Vakuum bei 60°C getrocknet. Man erhält 2,4 g der Titelverbindung, die mit 1 Mol Kristallwasser kristallisiert.

Analyse: $C_{16}H_{14}N_2O_4Na_2 \times 1H_2O$

| | C % | H % | N % | $\lambda$max [nm] | log$\varepsilon$ |
|---|---|---|---|---|---|
| ber.: | 53,04 | 4,42 | 7,73 | 330 | 4,31 |
| gef.: | 52,27 | 4,40 | 7,46 | 290 | 4,22 |

Beispiel 2

2-(5-Isopropylpyridyl-2-amino)-5-(1H-tetrazol-5-yl)-benzoesäuredinatriumsalz

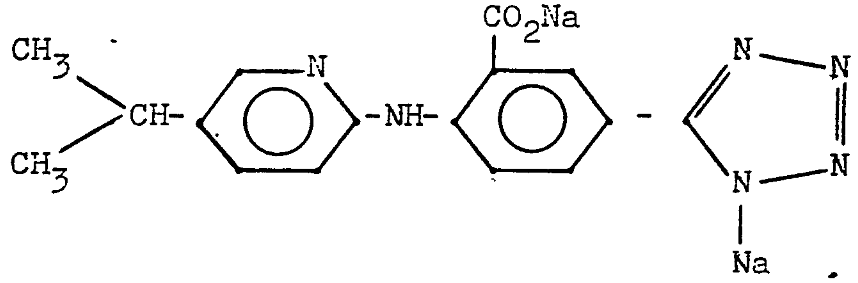

6,0 g 2-(5-Isopropylpyridyl-2-amino)5-cyanobenzoesäure natriumsalz, 1,8 g Natriumazid und 1,5 g Ammoniumchlorid werden in 70 ml Dimethylformamid 12 Stunden auf 100°C erhitzt. Die anorganischen Bestandteile werden dann abgetrennt und die Lösung eingedampft. Der Rückstand wird mit Ethanol-Ether verrieben, wobei die gewünschte Verbindung kristallisiert.

In analoger Weise werden folgende Verbindungen hergestellt:

N-Formyl-N-(5-Isopropylpyridyl-2)-4-aminoisopthalsäure-dinatriumsalz

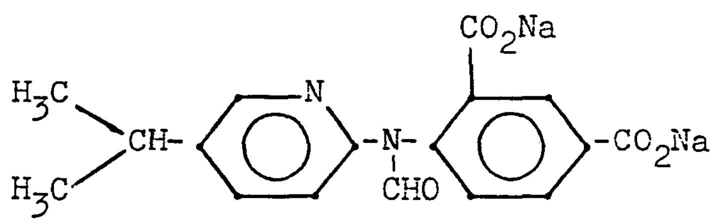

4-(5-Pentyl-3-pyridyl-2-amino)isophthalsäure-dinatriumsalz

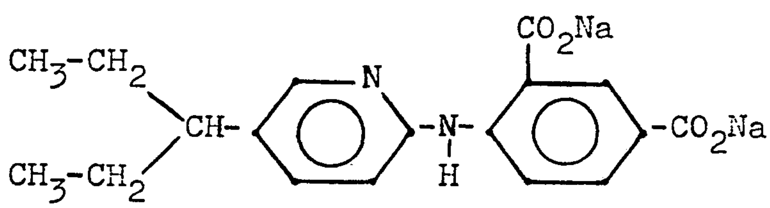

N-Acetyl-N-(5-Butyl-2-pyridyl-2)-4-aminoisophthalsäure-dinatriumsalz

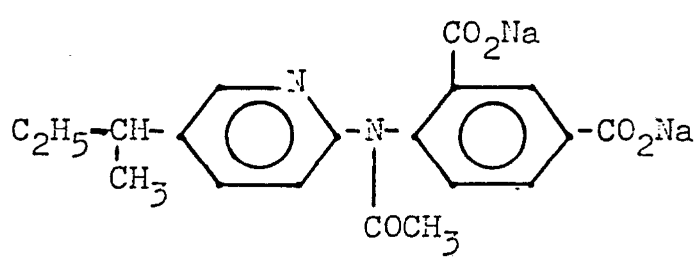

4-(5-Butyl-2-pyridyl-2-amino)-isophthalsäure-dikalium-salz

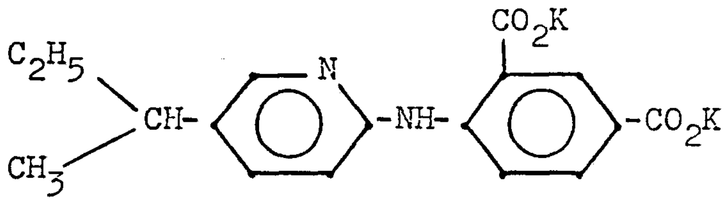

N-Formyl-N-(5-Isopropylpyridyl-2)-2-amino-5-(1H-tetrazol-5-yl)-benzoesäure-dikaliumsalz

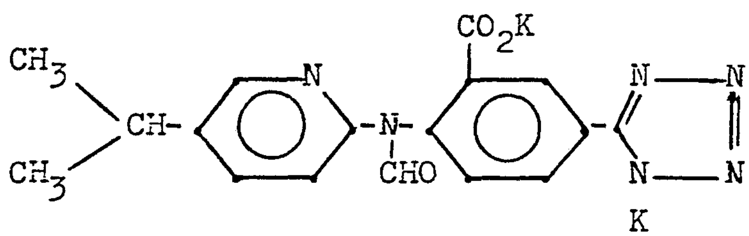

N-Acetyl-N-(5-Isopropylpyridyl-2-)-2-amino-5-(1H-tetrazol-5-yl)-benzoesäure-dikaliumsalz

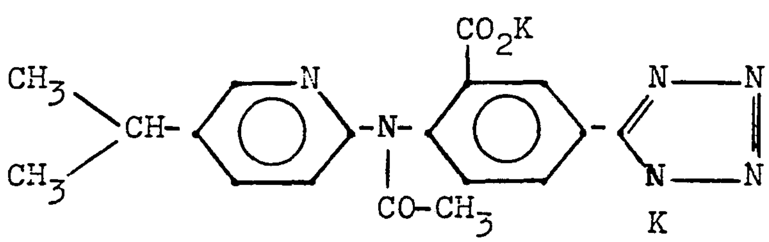

2-(5-Butyl-2-pyridyl-2-amino)-5-(1H-tetrazol-5-yl)-benzoesäure-dinatriumsalz

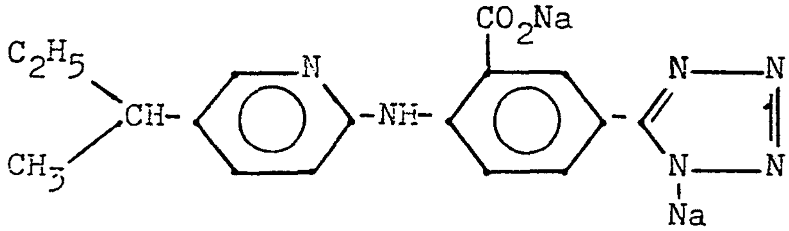

2-(5-Pentyl-3-pyridyl-2-amino)-5-(1H-tetrazol-5-yl)-benzoesäure-dinatriumsalz

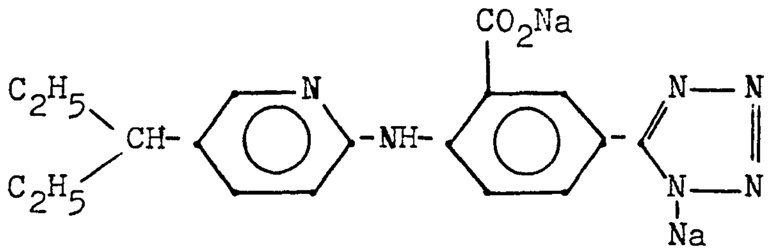

Patentansprüche

1. Verbindungen der Formel

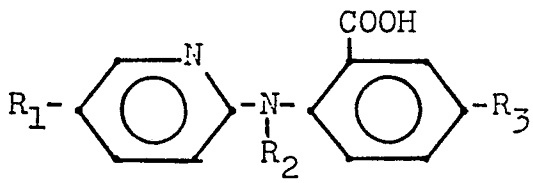 (I),

in der

$R_1$ für eine der Gruppen

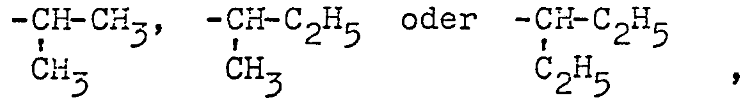

$R_2$ für Wasserstoff, -CHO oder $-CO-CH_3$ und

$R_3$ für COOH oder 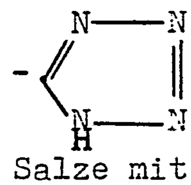

steht, sowie ihre Salze mit anorganischen oder organischen Basen.

2. Pharmazeutische Zubereitungen, gekennzeichnet durch einen Gehalt an einer Verbindung nach Anspruch 1.

3. Verwendung von Verbindungen nach Anspruch 1 bei der Prophylaxe oder Bekämpfung allergischer Krankheiten.

4. Verfahren zur Herstellung von Verbindungen nach Anspruch 1, dadurch gekennzeichnet, daß man

a) bei einer Verbindung der Formel

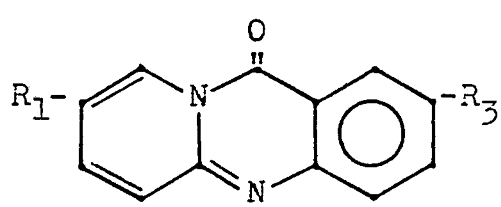 (II)

durch Behandlung mit basischen Stoffen eine Ringöffnung bewirkt und gewünschtenfalls anschließend die Aminogruppe formyliert oder acetyliert

oder daß man

b) bei einer Verbindung der Formel

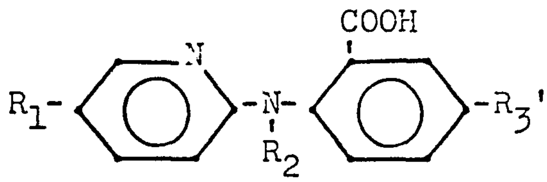

(III),

in der $R_3'$ eine Vorstufe von $R_3$ bedeutet, die Gruppe $R_3'$ in die Gruppe $R_3$ umwandelt oder daß man

c) aus einer Verbindung der Formel

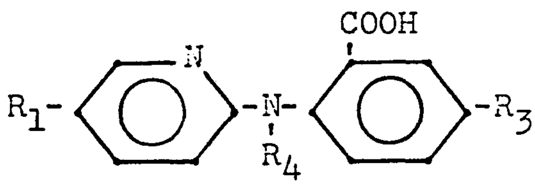

(IV),

in der $R_4$ eine von der Aminogruppe abspaltbare Gruppe bedeutet, die Gruppe $R_4$ abspaltet.

5. Verbindungen nach Anspruch 1, dadurch gekennzeichnet, daß $R_1$ für die Isopropylgruppe, $R_2$ für Wasserstoff und $R_3$ für die Carboxylgruppe steht, und ihre Salze mit anorganischen oder organischen Basen.

6. Verbindungen nach Anspruch 1, dadurch gekennzeichnet, daß $R_1$ für die Butyl-2-gruppe, $R_2$ für Wasserstoff und $R_3$ für die Carboxylgruppe steht.

7. Verbindungen nach Anspruch 1, dadurch gekennzeichnet, daß $R_1$ für die Pentyl-3-gruppe, $R_2$ für Wasserstoff und $R_3$ für die Carboxylgruppe steht.

8. Verbindungen nach Anspruch 1, dadurch gekennzeichnet, daß $R_1$ für die Isopropylgruppe, $R_2$ für Wasserstoff und $R_3$ für die Tetrazol-5-ylgruppe steht.

9. Verbindungen nach Anspruch 1, dadurch gekennzeichnet, daß $R_1$ für die Butyl-2-gruppe, $R_2$ für Wasserstoff und $R_3$ für die Tetrazol-5-ylgruppe steht.

10. Verbindungen nach Anspruch 1, dadurch gekennzeichnet, daß $R_1$ für die Pentyl-3-gruppe, $R_2$ für Wasserstoff und $R_3$ für die Tetrazol-5-ylgruppe steht.

Europäisches Patentamt

# EUROPÄISCHER RECHERCHENBERICHT

0056114

Nummer der Anmeldung

EP 81 11 0105.4

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| D,X | DE - A1 - 2 735 919 (C.H. BOEHRINGER )<br>* Ansprüche 1 bis 4 *<br>-- | 1-4 | C 07 D 213/74<br>C 07 D 401/12<br>A 61 K 31/44<br>//C 07 D 213/75 |
| A | US - A - 4 186 204 (R.G. HARRISON et al.)<br>* Ansprüche 1, 19, 22 *<br>& DE - A - 2 625 227<br>-- | 1-3 | |
| A | JOURNAL OF MEDICINAL CHEMISTRY, Band 14, Nr. 5, 1971<br>Washington<br>D.M. BAILEY et al. "Effect of 2-anilinopyridines on Protein Synthesis"<br>Seiten 439 bis 443<br>* Seite 440, Verbindung Nr. 7 *<br>---- | 1 | |

**RECHERCHIERTE SACHGEBIETE (Int. Cl.³)**

A 61 K 31/44
C 07 D 213/74
C 07 D 213/75
C 07 D 257/04
C 07 D 401/12

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 01-04-1982 | FROELICH |

EPA form 1503.1 06.78